# EUROPÄISCHE PATENTANMELDUNG

(11) **EP 1 704 898 A1**
(43) Veröffentlichungstag der Anmeldung: **27.09.2006**
(21) Anmeldenummer: 06003763.7
(22) Anmeldetag: 24.02.2006
(51) Int. Cl.: A61Q 19/00, A61Q 19/10, A61K 8/92

(54) **Oxidationsgeschützte kosmetische Zubereitung mit Reiskeimöl**

(30) Priorität: 24.03.2005 DE 102005014417
(71) Anmelder: Beiersdorf AG, 20253 Hamburg (DE)
(72) Erfinder: von der Fecht, Stephanie, 22880 Wedel (DE); Küther, Jörg, Dr., 25469 Halstenbek (DE); Liste, Kathrin, 22301 Hamburg (DE)

(57) **Zusammenfassung**

Kosmetische Zubereitung enthaltend Reiskeimöl.

## Beschreibung

Die vorliegende Erfindung betrifft den Schutz kosmetischer Formulierungen gegen oxidative Degradation und so geschützte Zubereitungen.
Oxidationsempfindliche Zubereitungen im Sinne der vorliegenden Schrift sind oxidationsemfindliche Öle besonders Pflanzenöle, die eine hohen Anteil an ungesättigten Fettsäuren haben. Dies wird insbesondere über die lodzahl charakterisiert. Im Sinne der Erfindung ist ein oxidationsempfindliches Öl ein Öl mit einer lodzahl größer als 80. Weitere Oxidationsempfindliche Substanzen im Sinn der Erfindung sind pflanzliche Extrakte, besonders bevorzugt sind hier die Extrakte der Pflanzenfamilien der Nymphaeaceae, Nelumbonaceae, Fabaceae und/oder Leguminosae.

### Stand der Technik

In Wasser enthaltenden kosmetischen Formulierungen werden häufig pflanzliche Öle oder Wirkstoffe eingesetzt, um dem Produkt eine pflegende Wirkung zu verleihen. Hierzu werden oftmals Pflanzenöle mit einem hohen Anteil an ungesättigten Fettsäuren eingesetzt, da diese den natürlichen Hautlipiden in Struktur und Zusammensetzung sehr nahe kommen und sehr gut von der Haut aufgenommen werden. Je mehr ungesättigte Einheiten in einem Öl vorhanden sind desto größer ist dessen Neigung durch oxidative Prozesse zu verderben. In kosmetischen Produkten führt dies häufig zu unerwünschten Farb-und Geruchsbeeinträchtigungen.
Bisher werden diese pflanzlichen Öle durch zusätzliche Stabilisatoren (wie BHT, Propyl Gallate, Tocopherol Acetate, EDTA, IDS, UV Filter, Benzophenone etc.) in der Formulierung stabilisiert. Viele dieser Stoffe sind problembehaftet und verursachen zudem erhebliche Mehrkosten.

Eine besondere Herausforderung besteht darin, einen alternativen Weg zur Langzeitstabilsierung dieser pflanzlichen Öle enthaltende Formulierung zu finden.

### Erfindung

Es wurden nun gefunden, daß die gravierenden Nachteile des Standes der Technik, insbesobndere die der mangelnden Farb- und Oxidationsstabilität vermieden werden können durch kosmetische Zubereitung enthaltend Reiskeimöl. So werden Geruchsbeeinträchtigungen und Qualitätsverminderungen von kosmetischen Zubereitungen in für den Fachmann nicht vorhersehbarer Weise überwunden.
Kosmetische Zubereitungen mit oxidationsempfindlichen Inhaltsstoffen werden für den Fachmann überraschend durch die Zugabe von Reisöl (Oryza sativa) stabilisiert. Insbesondere überrascht dies, da es sich bei dem Reisöl selbst um ein ungesättigtes Öl handelt. Reisölhaltige Zubereitungen eignen sich sowohl als Leave on Produkte wie Emulsionen, Körperöle, usw., als auch als Rinse off Produkte wie Dusch- und Badeprodukte, Shampoos, usw. Diese zeichnen sich dadurch aus, dass sie in weitem Maße in der Lage sind Verbrauchererwartungen zu erfüllen und dadurch eine beträchtliche Marktbedeutung aufweisen.
Dabei ist es bevorzugt, wenn der Gehalt an Reiskeimöl 0,05 bis 6 Gew.%, besonders bevorzugt 0,2 bis 5 Gew.%, ganz besonders bevorzugt 0,5 bis 2 Gew.% beträgt.
Dabei ist es bevorzugt, wenn zusätzlich eine oxidationsempfindliche Substanz enthalten ist.
Weiter ist es bevorzugt, wenn die oxidationsempfindliche Substanz in Konzentrationen von mindestens 0,1 Gew.%, besonders bevorzugt 0,5 Gew.%, ganz besonders bevorzugt 1 Gew.%, ganz besonders bevorzugt 5 Gew.% enthalten ist. Dabei ist es bevorzugt, wenn die oxidationsempfindliche Substanz eine Jodzahl von mindestens 80 aufweist. Weiter ist es bevorzugt, wenn die oxidationsempfindliche Substanz ein oxidierbares pflanzliches Öl oder einen pflanzlichen Extrakt, besonders bevorzugt Extrakte der Pflanzenfamilien der Nymphaeaceae, Nelumbonaceae, Fabaceae und/oder Leguminosae enthält. Dabei ist es bevorzugt, wenn Zubereitung mindestens 1 Gew.%, besonders bevorzugt mehr als 2 Gew.% oberflächenaktive Substanz, ganz besonders bevorzugt mit einem HLB Wert zwischen 3,8 bis 8 oder größer 10, ganz aussergewöhnlich besonders bevorzugt zwischen 5 und 7 oder größer 12 enthält.

Dabei ist es bevorzugt, wenn die oberflächenaktive Substanz aus der Gruppe ethoxylierter Fettalkoholderivate mit einer C-Kettenlänge größer 10 und einem mittleren Ethoxylierungsgrad zwischen 2 und 75, besonders bevorzugt zwischen 2 und 39 ganz besonders bevorzugt zwischen 2 und 5 gewählt wird.

Weiter ist es bevorzugt, wenn als oberflächenaktive Substanzen anionische Tenside, besonders bevorzugt Alkyl- und/oder Alkylethersulfate in Anteilen von 2-25 Gew.%, bevorzugt 3-20 Gew.% und besonders bevorzugt 7,5-15 Gew.% enthält.
Weiter ist es bevorzugt, wenn sie weitere anionische, amphotere oder nicht ionische Tenside von 1-15 Gew.%, besonders bevorzugt 3-10 Gew.% und ganz besonders bevorzugt 4-7 Gew.% enthält.
Dabei ist es bevorzugt, wenn sie
(a) 1 - 25 Gew.% eines oder mehrerer Alkyl- und/oder Alkylethersulfate,
(b) 0,1 -20 Gew.% N-Acylglutamat,
(c) wobei der Gehalt an freien Fettsäuren in den Zubereitungen geringer als 3 Gew.% bevorzugt geringer als 2 Gew. % besonders bevorzugt bis zu 1 Gew.%, aber in allen Fällen mindestens 0,2 Gew.% beträgt,
enthält.
Weiter ist es bevorzugt, wenn die Zubereitung einen Lipidanteil von mindestens 5 Gew.%, bevorzugt mindestens 10 Gew.%, besonders bevorzugt mindestens 15 Gew.% aufweist.
Durch weitere Zugabe geringer Mengen von Oryzanol wird ein synergistischer Effekt erzielt der sich in überraschender Weise sogar zu Stabilisierung besonders oxidationsempfindlicher Substanzen eignet.
Daher ist es bevorzugt, wenn die Zubereitung zusätzlich mehr als 0,007 Gew.% Oryzanol, bevorzugt mehr als 0,01 Gew.% Oryzanol enthält.
Die Erfindung umfasst auch die Verwendung von erfindungsgemäßen Zubereitungen zur Körperpflege und/oder Körperreinigung.
Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

### Vorteilhafte Ausführungsformen

Erfindungsgemäße Zubereitungen enthalten bevorzugt Tenside. Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und -je nach Wunsch- für Schaumregulierung.
Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.
Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische lonen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine lonen.

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Sarcosinate, beispielsweise Myristoyl Sarcosin, TEA-lauroyl Sarcosinat, Natriumlauroylsarcosinat und Natriumcocoylsarkosinat,
Carbonsäuren und Derivate, wie
1. Carbonsäuren, beispielsweise Laurinsäure, Aluminiumstearat, Magnesiumalkanolat und Zinkundecylenat,
   Sulfonsäuren und Salze, wie

1. Acyl-isethionate, z.B. Natrium-/ Ammoniumcocoyl-isethionat,
2. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
3. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkylamidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
7. Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden. Hier haben sich als besonders vorteilhaft folgende Tenside für den Einsatz erwiesen: Natriumacylglutamat, Natriumlauroylsarcosinat, Natriumisethionat, Dinatriumlaurylsulfosuccinat, Natriumlaurethsulfat, Natriumparethsulfat, Alkylamidopropylbetain, Natriumalkylamphodiacetat, Alkylpolyglycoside wie Laurylglucosid, Decylglycosid und Cocoglycosid.
Den erfindungsgemäßen Zubereitungen können Emulgatoren zugesetzt sein. Erfindungsgemäß können die Silikonemulgatoren vorteilhaft aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur: 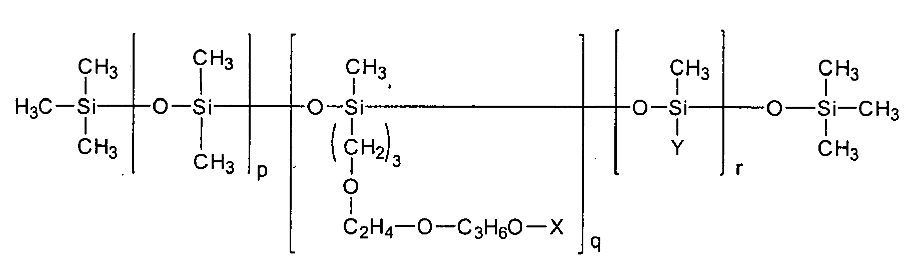 bei welcher X und Y unabhängig voneinander gewählt werden aus der Gruppe H (Wasserstoff) sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.

Ein Beispiel für besonders vorteilhaft im Sinne der vorliegenden Erfindung zu verwendende Silikonemulgatoren sind Dimethiconcopolyole, welche unter den Warenbezeichnungen ABIL® EM 90, ABIL® EM 97 und ABIL® B 88183 verkauft werden.

Der oder die W/O-Emulgatoren werden erfindungsgemäß vorzugsweise gewählt aus der folgenden Gruppe:
Sorbitanstearat, Sorbitanoleat, Lecithin, Glyceryllanolat, Lanolin, mikrokristallines Wachs (Cera microcristallina) im Gemisch mit Paraffinöl (Paraffinum liquidum), Ozokerit, hydriertem Ricinusöl, Glycerylisostearat, Polyglyceryl-3-Oleat, Wollwachssäuregemische, Wollwachsalkoholgemische, Pentaerythrithylisostearat, Polyglyceryl-3 Diisostearat, Sorbitan Oleat im Gemisch mit hydriertem Ricinusöl, Bienenwachs (Cera alba) und Stearinsäure, Natriumdihydroxycetylphosphat im Gemisch mit Isopropylhydroxycetylether, Methylglucosedioleat, Methylglucosedioleat im Gemisch mit Hydroxystearat und Bienenwachs, Mineralöl im Gemisch mit Petrolatum und Ozokerit und Glyceryloleat und Lanolinalkohol, Petrolatum im Gemisch mit Ozokerit und hydriertem Ricinusöl und Glycerylisostearat und Polyglyceryl-3-oleat, PEG-7-hydriertes Ricinusöl, Sorbitanoleat im Gemisch mit PEG-2-hydriertem Ricinusöl, Ozokerit und hydriertem Ricinusöl, Sorbitanisostearat im Gemisch mit PEG-2-hydriertem Ricinusöl, Polyglyceryl-4-isostearat, Polyglyceryl-4-isostearat, Hexyllaurat, Acrylat/ C₁₀₋₃₀-Alkylacrylat-Crosspolymer, Sorbitanisostearat, Poloxamer 101, Polyglyceryl-2-dipolyhydroxystearat, Polyglyceryl-3-Diisostearat, Polyglyceryl-4-dipolyhydroxystearat, PEG-30-dipolyhydroxystearat, Diisostearoylpolyglyceryl-3-diisostearat, Polyglyceryl-2-dipolyhydroxystearat, Polyglyceryl-3-dipolyhydroxystearat, Polyglyceryl-4-dipolyhydroxystearat, Polyglyceryl-3-dioleat.

Vorteilhaft werden die Emulgatoren gewählt aus der Gruppe der Sorbitanester und Saccharoseester, insbesondere der verzweigten und unverzweigten Alkylester und Alkenylester mit Kohlenstoffketten von 4 - 24 Kohlenstoffatomen, bevorzugt Sorbitanstearat, Sorbitanoleat, Glycerylsorbitanstearat, Saccharosemonostearat, Saccharosemonolaurat, Saccharosepalmitat.

Vorteilhaft können die Emulgatoren aus der Gruppe der Monoglycerin-monocarbonsäure-monoester gewählt werden, insbesondere solche, welche durch die Strukturen 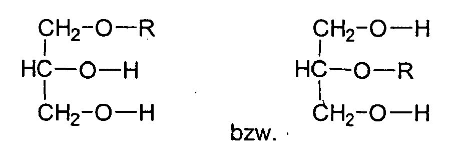 gekennzeichnet sind, wobei R' einen verzweigten oder unverzweigten Acylrest mit 6 - 14 Kohlenstoffatomen darstellt. Vorteilhaft wird R' gewählt aus der Gruppe der unverzweigten Acylreste.

Die diesen Estern zugrundeliegenden Säuren sind die

| | | |
|---|---|---|
| Hexansäure | (Capronsäure) | (R'=-C₅H₁₁), |
| Heptansäure | (Önanthsäure) | (R' = -C₆H₁₃), |
| Octansäure | (Caprylsäure) | (R' = -C₇H₁₅), |
| Nonansäure | (Pelargonsäure) | (R' = -C₈H₁₇), |
| Decansäure | (Caprinsäure) | (R' = -C₉H₁₉), |
| Undecansäure | | (R' = -C₁₀H₂₁), |
| 10-Undecensäure | (Undecylensäure) | (R' = -C₁₀H₁₉), |
| Dodecansäure | (Laurinsäure) | (R' = -C₁₁H₂₃), |
| Tridecansäure | | (R' = -C₁₂H₂₅), |
| Tetradecansäure | (Myristinsäure) | (R' = -C₁₃H₂₇), |

Vorteilhaft können die Emulgatoren auch aus der Gruppe der Di- und Triglycerin-monocarbonsäure-monoester gewählt werden. Erfindungsgemäß liegen die Di- bzw. Triglycerineinheiten der erfindungsgemäßen Diglycerin-monocarbonsäure-monoester bzw. Triglycerin-monocarbonsäure-monoester als lineare, unverzweigte Moleküle, also über die jeweiligen OH-Gruppen in 1- bzw. 3-Stellung veretherte "Monoglycerinmoleküle" vor.

Die erfindungsgemäßen Monocarbonsäuremonoester sind bevorzugt durch folgende Struktur gekennzeichnet: 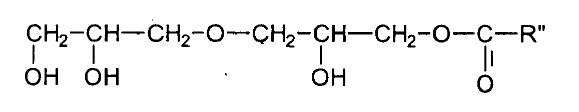 wobei R" einen Kohlenwasserstdffrest, vorteilhaft einen verzweigten oder unverzweigten Alkyl- oder Alkenylrest von 5 bis 17 C-Atomen darstellt.

Die erfindungsgemäßen Monocarbonsäureester des Triglycerins sind bevorzugt durch folgende Struktur gekennzeichnet: 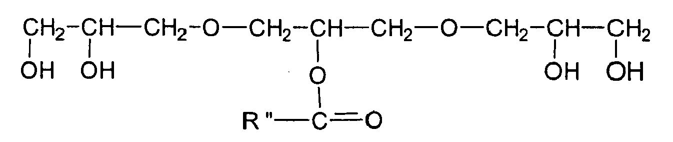 wobei R"' einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrunde liegenden Säuren sind die

| | | |
|---|---|---|
| Hexansäure | (Capronsäure) | (R" bzw.R"' = -C₅H₁₁), |
| Heptansäure | (Önanthsäure)' | (R" bzw. R"' = -C₆H₁₃), |
| Octansäure | (Caprylsäure) | (R" bzw. R'" = -C₇H₁₅), |
| Nonansäure | (Pelargonsäure) | (R" bzw. R'" = -C₈H₁₇), |
| Decansäure | (Caprinsäure) | (R" bzw. R"' = -C₉H₁₉), |
| Undecansäure | | (R" bzw. R'" = -C₁₀H₂₁), |
| 10-Undecensäure | (Undecylensäure) | (R" bzw. R"' = -C₁₀H₁₉), |
| Dodecansäure | (Laurinsäure) | (R" bzw. R"' = -C₁₁H₂₃), |
| Tridecansäure | | (R" bzw. R'" = -C₁₂H₂₅), |
| Tetradecansäure | (Myristinsäure) | (R" bzw. R'" = -C₁₃H₂₇), |
| Pentadecansäure | | (R" bzw. R'" = -C₁₄H₂₉), |
| Hexadecansäure | (Palmitinsäure) | (R" bzw. R"' = -C₁₅H₃₁), |
| Heptadecansäure | (Margarinsäure) | (R" bzw. R'" = -C₁₆H₃₃), |
| Octadecansäure | (Stearinsäure) | (R" bzw. R'" = -C₁₇H₃₅). |

Besonders günstig werden R" und R"' gewählt aus der Gruppe der unverzweigten Alkylreste mit ungeraden C-Zahlen, insbesondere mit 9, 11 und 13 C-Atomen.

Vorteilhaft sind auch Triglyceryldüsostearat (Nomenklatur analog CTFA: Polyglyceryl-3-diisostearat), Isostearyldiglycerylsuccinat, Diglycerylsesquüsostearat (Nomenklatur analog CTFA: Polyglyceryl-2-sesquüsostearat), Triglycerylpolyhydroxystearat (Nomenklatur analog CTFA: Polyglyceryl-2-polyhydroxystearat).

Ebenfalls besonders vorteilhaft werden der oder die Emulgatoren gewählt aus der Gruppe der mono-, oligo und polyethoxylierten Verbindungen, insbesondere der polyethoxylierten ein- oder mehrbasigen Alkohole oder Fettsäuren, beispielsweise Ceteareth-20, PEG-20-Glycerylstearat, Steareth-20, PEG-20-Stearat, PEG-30-Stearat, PEG-40-Stearate, PEG-40-Rizinusöl, PEG-1-Glycerin-Sorbitan-Oleostearat, PEG-7 hydriertes Rizinusöl, PEG-40-Sorbitanperoleat, PEG-45-Dodecyl-Glycol-Copolymer.

Erfindungsgemäße Zubereitungen enthalten bevorzugt als Lipide polare Öle. Polare Öle sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl (Jodzahl 77-90), Sonnenblumenöl (Jodzahl 122-138), Sojaöl (Jodzahl 124-132), Erdnußöl (Jodzahl 85-105), Rapsöl (Jodzahl 95-120), Mandelöl (Jodzahl 93-106), Palmöl, Kokosöl, Rizinusöl (Jodzahl82-90), Weizenkeimöl (Jodzahl 125-135), Traubenkernöl 125-142), Distelöl (Jodzahl 86-119), Nachtkerzenöl (Jodzahl 145-162), Macadamianußöl (Jodzahl 74-76) und dergleichen mehr.
Besonders vorteilhafte polare Lipide im Sinne der vorliegenden Erfindung sind alle nativen Lipide, wie z. B. Olivenöl (Jodzahl 77-90), Sonnenblumenöl (Jodzahl 122-138), Sojaöl (Jodzahl 124-132), Erdnußöl (Jodzahl 85-105), Rapsöl (Jodzahl 95-120), Mandelöl (Jodzahl 93-106), Palmöl, Kokosöl, Rizinusöl (Jodzahl82-90), Weizenkeimöl (Jodzahl 125-135), Traubenkernöl 125-142), Distelöl (Jodzahl 86-119), Nachtkerzenöl (Jodzahl 145-162), Macadamianußöl (Jodzahl 74-76), Maiskeimöl (Jodzahl 103-130), Avocadoöl (Jodzahl 80-95) und dergleichen sowie die im folgenden aufgelisteten.

| Hersteller | Handelsname | INCl-Name |
|---|---|---|
| Condea Chemie | Isofol 14 T | Butyl Decanol (+) Hexyl Octanol (+) Hexyl Decanol (+) Butyl Octanol |
| Lipochemicals INC. / USA (Induchem) | Lipovol MOS-130 | Tridecyl Stearate(+) Tridecyl Trimellitate(+) Dipentaerythrityl Hexacaprylate/Hexacaprate |
| Huels CONDEA Chemie | Miglyol 840 | Propylene Glycol Dicaprylate/Dicaprate |
| Goldschmidt | Tegosoft SH | Stearyl Heptanoate |
| Henkel Cognis | Cetiol B | Dibutyl Adipate |
| Condea Augusta S.P.A. | Cosmacol ELI | C12-13 Alkyl Lactate |
| Henkel Cognis | Myritol 331 | Cocoglycerides |
| Unichema | Prisorine 2041 GTIS | Triisostearin |

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase einen Gehalt an C₁₂₋₁₅-Alkylbenzoat aufweist oder vollständig aus diesem besteht.
Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 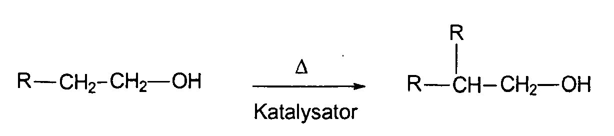 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemittein eingesetzt werden.
Die Verwendung von Guerbet-Alkoholen in Kosmetika ist an sich bekannt. Solche Species zeichnen sich dann meistens durch die Struktur 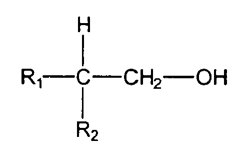 aus. Dabei bedeuten R₁, und R₂ in der Regel unverzweigte Alkylreste.
Erfindungsgemäß vorteilhaft werden der oder die Guerbet-Alkohole gewählt aus der Gruppe, bei denen
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.
Erfindungsgemäß bevorzugte Guerbet-Alkohole sind das 2-Butyloctanol - es hat die chemische Struktur 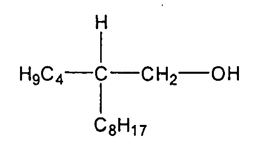 und ist beispielsweise unter der Handelsbezeichnung Isofol® 12 von der Gesellschaft Condea Chemie GmbH erhältlich - und das 2-Hexyldecanol - es hat die chemische Struktur 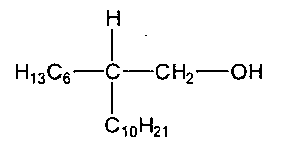 und ist beispielsweise unter der Handelsbezeichnung Isofol® 16 von der Gesellschaft Condea Chemie GmbH erhältlich. Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 von der Gesellschaft Condea Chemie GmbH erhältlich.

Besonders vorteilhafte Lipide im Sinne der vorliegenden Erfindung sind die im folgenden aufgelisteten Substanzen:

| Hersteller | Handelsname | INCI-Name |
|---|---|---|
| Stearinerie Dubois Fils | DUB VCI 10 | Isodecyl Neopentanoate |
| Henkel Cognis | Cetiol SN | Cetearyl Isononanoate |
| Unichema | Isopropylpalmitat | Isopropylpalmitat |
| Henkel Cognis | Eutanol G | Octyldodecanol |
| Bayer AG, Dow Corning | Silikonöl VP 1120 | Phenyl Trimethicone |
| Henkel Cognis | Isopropylstearat | Isopropyl Stearate |
| WITCO, Goldschmidt | Finsolv TN | C12-15 Alkyl Benzoate |
| Dr. Straetmans | Dermofeel BGC | Butylene Glycol Caprylate/Caprate |
| Unichema Huels | Miglyol 812 | Caprylic/Capric Triglyceride |

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

| Hersteller | Handelsname Handelsname | INCl-Name |
|---|---|---|
| Neste PAO N.V. | | Polydecene |
| (Lief. Hansen & Rosenthal) | Nexbase 2006 FG | |
| EC Erdölchemie (Lieferant Bayer AG) | Solvent ICH | Isohexadecane |
| DEA Mineralöl (Lief. Hansen & Rosenthal) | Pionier 2076 | Mineral Oil |
| Tudapetrol | | |
| DEA Mineralöl (Lief. Hansen & Rosenthal) | Pionier 6301 | Mineral Oil |
| Tudapetrol | | |
| EC Erdölchemie GmbH | Isoeikosan | Isoeikosan |
| DEA Mineralöl (Lief. Hansen & Rosenthal) | Pionier 2071 | Mineral Oil |
| Tudapetrol | | |
| Henkel Cognis | Cetiol CC | Dicaprylyl Carbonate |
| Henkel Cognis | Cetiol OE | Dicaprylyl Ether |
| Unichema | Estol 1540 EHC | Octyl Cocoate |

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

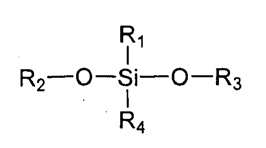

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 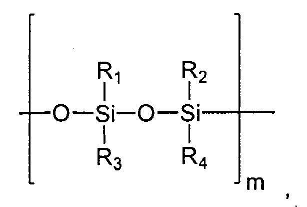 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 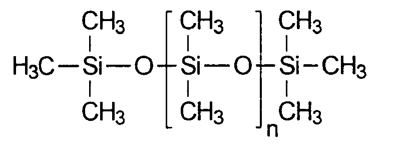 werden auch als Polydimethylsiloxan bzw. Dimethicon (INCl) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

| Hersteller | Handelsname | NCI-Name |
|---|---|---|
| Wacker | Wacker Silikonöl AK 100 | Polydimethylsiloxan |
| Dow Corning | Dow Corning Fluid 245 | Cyclopentasiloxan |
| Dow Corning | Dow Corning Fluid 345 | Cyclomethicone |

Erfindungsgemäß vorteilhaft einzusetzende cyclische Silicone werden im allgemeinen durch Strukturelemente charakterisiert, wie folgt 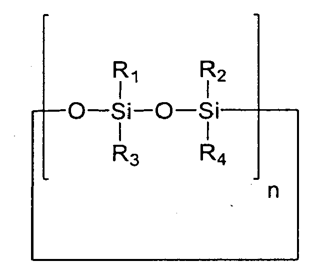 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Die erfindungsgemäßen Zubereitungen können weiterhin kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Filmbildner, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.
Die Menge der Verdickungsmittel beträgt beispielsweise 0,05 bis 5,0 Gew.-%, vorzugsweise 0,1 bis 3,0 Gew.-%, insbesondere 0,15 bis 2,0 Gew.-%, jeweils bezogen auf das Gesamtgewicht der Zubereitung.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.
Zur Bestimmung der lodzahl wird die Mehtode "lodzahl nach Wijs" (Cyclohexan / Eisessig Methode) herangezogen.

### Beispiele

### O/W-Emulsion mit Reisöl und Oryzanol

| | **1** | **2** | **3** | **4** | **5** |
|---|---|---|---|---|---|
| Glycerylsterat | 1,0 | --- | -- | 0,5 | 0,25 |
| Polyethylenglycol(40)stearat | 10,0 | --- | 1 | 5 | --- |
| Triglycerinmethylglucosedistearat | --- | 5,5 | --- | --- | 2,5 |
| Sorbitanstearat | --- | 1,5 | 2 | --- | --- |
| Cyclomethicon | 12,5 | 5 | --- | 10,0 | 3 |
| Dimethicon | 5,0 | 13,0 | 3,0 | 10,0 | 15,0 |
| Cetylalkohol | 2 | --- | 3 | 1 | --- |
| Stearylalkohol | --- | 1 | --- | 1 | --- |
| Cetylstearylalkohol | --- | --- | 1 | 1 | --- |
| C13-16 Isoparaffin | 0,5 | 1 | 3 | 2,0 | --- |
| Mineraloel | 0,5 | 1,0 | 6,0 | 3,0 | --- |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Traubenkernöl | 0,1 | | 0,25 | | 0,5 |
| Pflanzenextrakte | | 0,5 | | 0,01 | |
| Nachtkerzenöl | 0,2 | | 0,5 | | 0,25 |
| Jojobaöl | | 1,0 | | 0,2 | |
| Glycerin | 5 | 10 | 10 | 15 | 7,5 |
| Oryzanol | 0,025 | 0.05 | | 0.25 | 0.03 |
| Reizkeimöl | 5 | 0.5 | 0.2 | 1 | 0.75 |
| Wasser | ad 100 | ad 100 | ad 100 | ad 100 | ad 100 |

| | **6** | **7** | **8** | **9** | **10** |
|---|---|---|---|---|---|
| Polyethylenglycol(21)stearylether | 1 | -- | 2,5 | 2 | 1,5 |
| Polyethylenglycol(2)stearylether | 1 | -- | 5,5 | 3 | 7,5 |
| Cetearylglucosid | --- | 8 | --- | --- | --- |
| Cyclomethicon | 12,5 | 15 | 28,0 | 25,0 | 17,5 |
| Dimethicon | 5,0 | 13,0 | 5,0 | 12,0 | 15,0 |
| Behenylalkohol | 3 | 2 | --- | 1 | --- |
| Stearylalkohol | 3 | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Mineraloel | 5 | 10 | 15 | 3 | 7,5 |
| Glycerin | 5 | 10 | 15 | 3 | 7,5 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| modifizierte Stärke | 0,5 | --- | --- | 0,15 | -- |
| Traubenkernöl | 0,1 | | 0,25 | | 0,5 |
| Mandelöl | | 0,5 | | 0,01 | |
| Nachtkerzenöl | 0,2 | | 0,5 | | |
| Jojobaöl | | 1,0 | | 0,2 | 0,1 |
| Oryzanol | 0.025 | | 0.01 | 0.2 | |
| Reiskeimöl | 5 | 0.5 | 0.2 | 1 | 0.75 |
| Wasser | ad 100 | ad 100 | ad 100 | ad 100 | ad 100 |

| | **11** | **12** | **13** | **14** | **15** |
|---|---|---|---|---|---|
| Glycerylsteratcitrat | 1,0 | 0,5 | 0,1 | 0,5 | 0,3 |
| Polyethylenglycol(20)cetearylether | 10,0 | 1,0 | 5 | -- | -- |
| Triglycerinmethylglucosedistearat | --- | --- | --- | --- | 2,5 |
| Cyclomethicon | -- | --- | -- | 1 | -- |
| Dimethicon | 0,5 | 3,0 | 0,75 | 1,5 | 0,2 |
| Behenylalkohol | 1 | --- | 2 | 1 | 0,2 |
| Dicaprylylcarbonat | 3 | 5 | 10 | 15 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Panthenol | 0,5 | --- | 0,75 | 0,25 | 0,1 |
| Carbomer | 0,05 | 0,35 | 0,15 | 0,1 | --- |
| Parfum | q,s, | q,s, | q,s, | q,s, | q,s, |
| Caprylic/Capric Triglycerid | 1 | 5 | 3 | 5 | 10 |
| Methylparaben | 0,4 | 0,3 | 0,05 | 0,3 | 0,4 |
| Propylparaben | 0,3 | --- | 0,25 | 0,15 | --- |
| Iodopropynylbutylcarbamat. | --- | --- | 0,05 | --- | 0,1 |
| Phenoxyethanol | --- | 0,5 | --- | 0,15 | --- |
| Sorbitol | 10 | --- | -- | 5 | --- |
| Butylenglykol | --- | --- | --- | 5 | 10 |
| Propylenglykol | --- | --- | 10 | 5 | --- |
| Traubenkernöl | 0,1 | | 0,25 | | 0,5 |
| Mandelöl | | 0,5 | | 0,01 | |
| Nachtkerzenöl | 0,2 | | 0,5 | | |
| Jojobaöl | | 1,0 | | 0,2 | 0,1 |
| Pflanzenextrakte | 0,1 | | 0,15 | | 0,3 |
| Glycerin | --- | 7,5 | --- | --- | --- |
| Oryzanol | | 0.05 | 0.01 | 0.25 | 0.03 |
| Reiskeimöl | 5 | 0.5 | 0.2 | 1 | 0.75 |
| Wasser | ad 100 | ad 100 | ad 100 | ad 100 | ad 100 |

| | **16** | **17** | **18** | **19** | **20** |
|---|---|---|---|---|---|
| Glycerylsteratcitrat | 1,0 | 0,75 | 0,1 | 0,5 | 0,3 |
| Polyethylenglycol(20)cetearylether | 1,0 | --- | 2 | 1,5 | -- |
| Triglycerinmethylglucosedistearat | --- | 5,5 | --- | --- | 2,5 |
| Cyclomethicon | 2 | 4 | 6 | 1 | 3 |
| Dimethicon | -- | 0,5 | 0,75 | --- | --- |
| Behenylalkohol | 1 | --- | 2 | 1 | 0,2 |
| Stearylalkohol | --- | 1 | --- | 1 | 0,2 |
| Cetylstearylalkohol | --- | --- | 1 | 1 | 0,2 |
| Mineralöl | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Carbomer | 0,05 | 0,1 | 0,15 | 0,1 | --- |
| Parfum | q,s, | q,s, | q,s, | q,s, | q,s, |
| Dicaprylylcarbonat | 3 | 5 | 10 | 15 | 5 |
| Tocopherol | 0,5 | 1,0 | 0,75 | 0,25 | 0,1 |
| Caprylic/Capric Triglycerid | 1 | 2 | 3 | 5 | 10 |
| Methylparaben | 0,4 | 0,1 | 0,25 | 0,3 | 0,4 |
| Phenonip | 0,3 | 0,4 | 0,25 | 0,15 | --- |
| modifizierte Stärke | --- | 2,5 | --- | 0,15 | --- |
| Traubenkernöl | 0,1 | | 0,25 | | 0,5 |
| Mandelöl | | 0,5 | | 0,01 | |
| Pflanzenextrakte | | 0,3 | | | 0,4 |
| Nachtkerzenöl | 0,2 | | 0,5 | | |
| Jojobaöl | | 1,0 | | 0,2 | 0,1 |
| Glycerin | 3 | 5 | 8 | 12 | 10 |
| Oryzanol | 0.025 | | 0.01 | | 0.03 |
| Reiskeimöl | 5 | 0.5 | 0.2 | 1 | 0.75 |
| Wasser | ad 100 | ad 100 | ad 100 | ad 100 | ad 100 |

### WO/Emulsionen

| | **1** | **2** | **3** | **4** | **5** |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,4 | 0,2 | 0,4 | 0,6 | 2,0 |
| Traubenkernöl | 0,1 | | 0,25 | | 0,5 |
| Mandelöl | | 0,5 | | 0,01 | |
| Nachtkerzenöl | 0,2 | | 0,5 | | |
| Pflanzenextrakte | | 1,0 | | 0,2 | 0,1 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Benzylalkohol | 0,3 | 0,4 | 0,25 | 0,15 | --- |
| Dehydracetsäure, Na-Salz | --- | --- | 0,05 | --- | 0,1 |
| Orizanol | 0.025 | 0.05 | 0.01 | 0.25 | 0.03 |
| Reiskeimöl | 5 | 0.5 | 0.2 | 1 | 0.75 |
| Wasser | ad 100 | ad 100 | ad 100 | ad 100 | ad 100 |

### W/S-Emulsionen

| | **11** | **12** | **13** | **14** | **15** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon | 10,0 | 12,5 | 15,0 | -- | -- |
| Cyclomethicon | 12,5 | 15 | 18,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | --- | 1,75 | 0,3 | 1,0 |
| Natriumlactat | 0,4 | --- | 3,5 | 0,6 | 2,0 |
| Traubenkernöl | 0,1 | | 0,25 | | 0,5 |
| Mandelöl | | 0,5 | | 0,01 | |
| Nachtkerzenöl | 0,2 | | 0,5 | | |
| Pflanzenextrakte | 0,2 | | | 0, 5 | |
| Jojobaöl | | 1,0 | | 0,2 | 0,1 |
| Parfum | q,s, | q,s, | q,s, | q,s, | q,s, |
| Kaliumsorbat | 0,4 | 0,1 | 0,05 | 0,3 | 0,5 |
| C5-Cyclomethicon | 1,0 | --- | 7,0 | --- | --- |
| lodopropynylbutylcarbamat | --- | --- | 0,05 | --- | 0,1 |
| Orizanol | 0.025 | 0.05 | 0.01 | 0.25 | 0.03 |
| Reiskeimöl | 5 | 0.5 | 0.2 | 1 | 0.75 |
| Wasser | ad 100 | ad 100 | ad 100 | ad 100 | ad 100 |

### W/O-Emulsionen

| | **21** | **22** | **23** | **24** | **25** |
|---|---|---|---|---|---|
| PEG-22 Dodecyl Glycol Copolymer | 5,0 | 1,5 | 0,25 | --- | 3,0 |
| PEG-45 Dodecyl Glycol Polymer | 1,0 | 1,5 | 1,75 | 3,0 | -- |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Nachtkerzenöl | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Benzylalkohol | --- | --- | --- | 1,0 | 0,75 |
| Kaliumsorbat | 0,4 | 0,15 | 0,05 | 0,3 | 0,4 |
| Pflanzenextrakte | 0,2 | | | 0,3 | |
| Orizanol | 0.025 | 0.05 | 0.01 | 0.25 | 0.03 |
| Reiskeimlöl | 5 | 0.5 | 0.2 | 1 | 0.75 |
| Wasser | ad 100 | ad 100 | ad 100 | ad 100 | ad 100 |

### W/O-Emulsionen

| | **26** | **27** | **28** | **29** | **30** |
|---|---|---|---|---|---|
| Polyglyceryl-2-dipolyhydroxystearat | 3,0 | --- | 0,25 | --- | 3,0 |
| Polyglyceryl-3 diisostearate | 1,0 | 3,5 | 1,75 | 2,5 | -- |
| PEG-40 sorbitanisostearate | --- | 2,5 | 0,5 | 3,5 | 3,0 |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| Kaliumsorbat | 0,05 | 0,1 | 0,5 | 0,75 | 0,25 |
| hydriertes Rizinusöl | 0,5 | 0,75 | 1,0 | 2,5 | 5,0 |
| Talkum, micronisiert | 0,5 | 1,0 | --- | 0,25 | 0,1 |
| Pflanzenextrakte | 0,2 | | | 0,3 | |
| Traubenkernöl | 0,1 | | 0,25 | | 0,5 |
| Mandelöl | | 0,5 | | 0,01 | |
| Nachtkerzenöl | 0,2 | | 0,5 | | |
| Jojobaöl | | 1,0 | | 0,2 | 0,1 |
| Orizanol | 0.025 | 0.05 | 0.01 | 0.25 | 0.03 |
| Reiskeimöl | 5 | 0.5 | 0.2 | 1 | 0.75 |
| Wasser | ad 100 | ad 100 | ad 100 | ad 100 | ad 100 |

### Bath Care Formulierungen:

### Folgende Stoffe wurden eingesetzt:

- Polyacrylat:: Carbopol ETD 2020, Noveon
- Acrylates Copolymer:: Carbopol Aqua SF-1 Polymer, Noveon
- Natriumlaurethsulfat:: Hier wurde Texapon N 70 von der Firma Cognis verwendet.
Gleiche Ergebnisse werden auch mit den Rohstoffen Genapol LRO Paste von Clariant und Emal 270 D/B von Kao erzielt.
- Cocamidopropylbetain:: Tego-Betain F 50, Goldschmidt
- Natrium Myristylethsulfat:: Texapon K 14 Spezial 70%, Cognis
- Alkylpolyglucosid:: Hier kam Plantacare 2000 UP der Firma Cognis zum Einsatz. Ähnliche Ergebnisse werden auch mit Plantacare 1200 UP ebenfalls von Cognis erreicht.
- Natriumcocoylglutamat:: Plantapon ACG-35, Cognis
- Natrium Cocoamphoacetat:: Rewoteric AM C, Goldschmidt Rewo
- PEG-7 Glyceryl Cocoate: Tegosoft GC von Goldschmidt kommt hier zum Einsatz. Auch Cetiol HE von Cognis und Glycerox HE B von Croda können eingesetzt werden.
PEG-40 hydriertes Ricinusöl:Emulgin HRE, Cognis
PEG-200 hydriertes Glycerylpalmitat: Rewoderm LI 520-70, Goldschmidt Rewo
PEG-90 Glyceryl Isostearat: Oxetal VD 92, Zschimmer&Schwarz

**Die in der Rezeptur angegebenen Gehalte sind die Aktivgehalte der jeweiligen Rohstoffe**.

### Duschen

| | **1** | **2** | **3** | **4** | **5** |
|---|---|---|---|---|---|
| Natrium Laurethsulfat | 11,0 | 5,0 | 9,5 | 9,5 | 8,5 |
| Natrium Myrethsulfat | | | 3,0 | | |
| Cocoamidopropylbetain | 3,5 | 4,5 | 3,0 | 3,5 | 4,0 |
| Natriumcocoylglutamat | 1,5 | | 1,5 | 2,5 | 2,5 |
| Decyl Glucoside | | | | | 1,5 |
| PEG-40 hydriertes Rizinusöl | 0,5 | 0,5 | 0,5 | 0,5 | 0,5 |
| PEG-7 Glyceryl Cocoat | 2,0 | 2,3 | 2,0 | 1,5 | |
| Glycerin | | | 0,5 | | 1,0 |
| PEG-200 hydriertes Glycerylpalmitat | 0,5 | | 0,5 | 0,1 | 0,3 |
| PEG-90 Glyceryl Isostearat | | 0,3 | | 0,5 | |
| PEG-120 Methyl Glucose Dioleat | | 0,2 | | | |
| Traubenkernöl | 0,1 | | 0,5 | | 0,1 |
| Mandelöl | | 0,1 | | 0,25 | |
| Nachtkerzenöl | | 0,25 | | | |
| Reiskeimöl | 0,25 | 0,2 | 0,5 | 0,75 | 1,0 |
| Jojobaöl | | 0,2 | | | 0,25 |
| Laureth-2 | | 0,1 | | 0,1 | |
| Natriumchlorid | 1,0 | 1,0 | 2,0 | 1,0 | 1,0 |
| Trinatrium EDTA | 0,2 | | 0,2 | | 0,2 |
| Tetranatrium Imminodisuccinat | | 0,8 | | | |
| Polyquaternium-10 | 0,2 | | | 0,2 | |
| Guar Hydroxypropyl Trimonium Chlord | | | | 0,3 | |
| Benzophenone-4 | | | 0,1 | | 0,1 |
| Glycol Distearat | | 0,6 | | | |
| Glycerin | | 0,3 | | | |
| Laureth-4 | | 0,3 | | | |
| Styrene/Acrylate Copolymer | 1,0 | | | 1,0 | |
| Alcohol denat. | | 1,0 | | | |
| Pflanzenextrakte | | | | 0,2 | |
| Oryzanol | 0,05 | | 0,1 | 0,5 | |
| Konservierungsstoffe | q.s. | q.s. | q.s. | q.s. | q.s. |
| Farbstoffe | q.s. | q.s. | q.s. | q.s. | q.s. |
| Citronensäure | q.s. | q.s. | q.s. | q.s. | q.s. |
| Parfum | q.s. | q.s. | q.s. | q.s. | q.s. |
| Wasser | ad | ad | ad | ad | ad |

### Peeling-Produkte

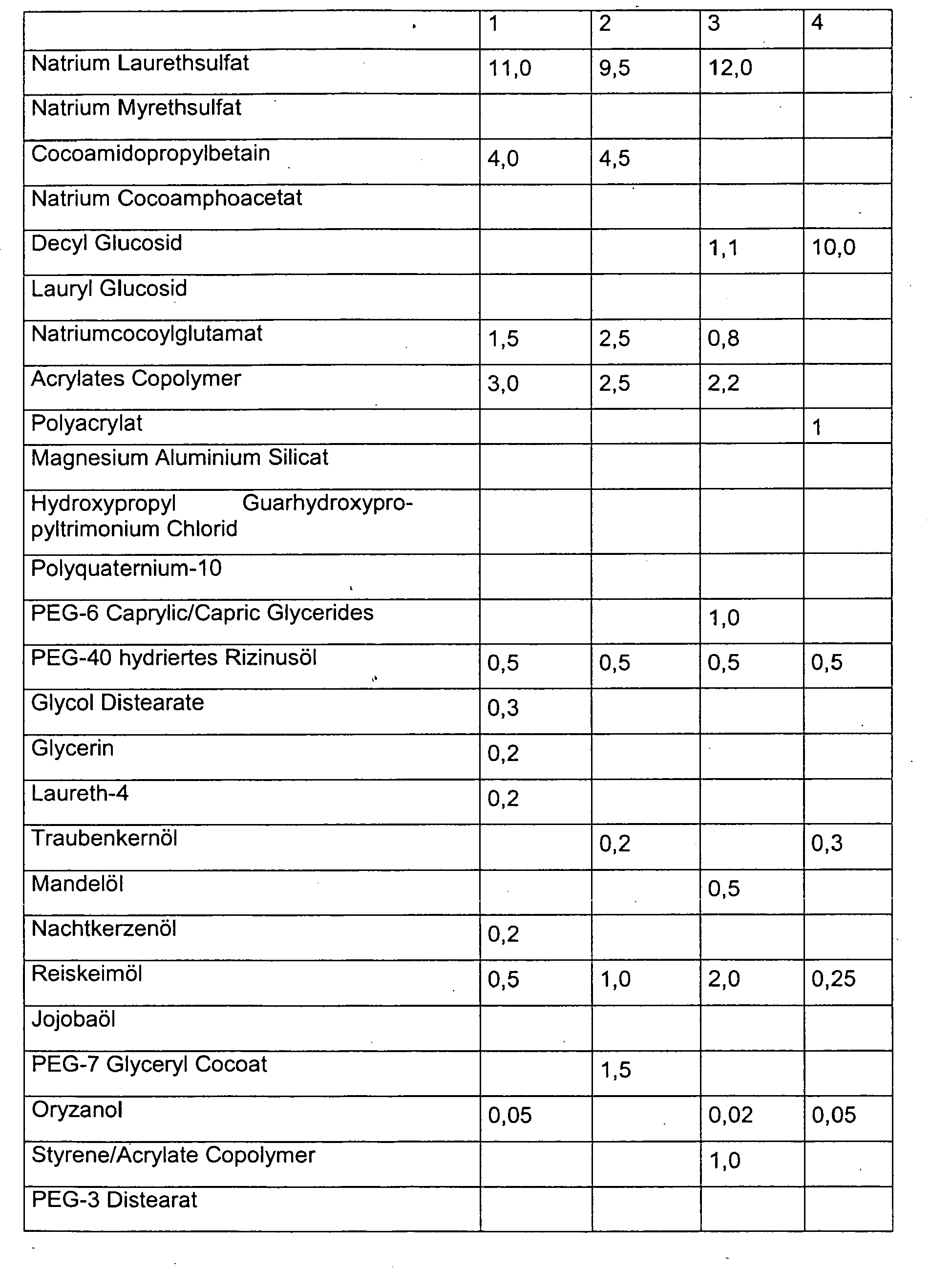

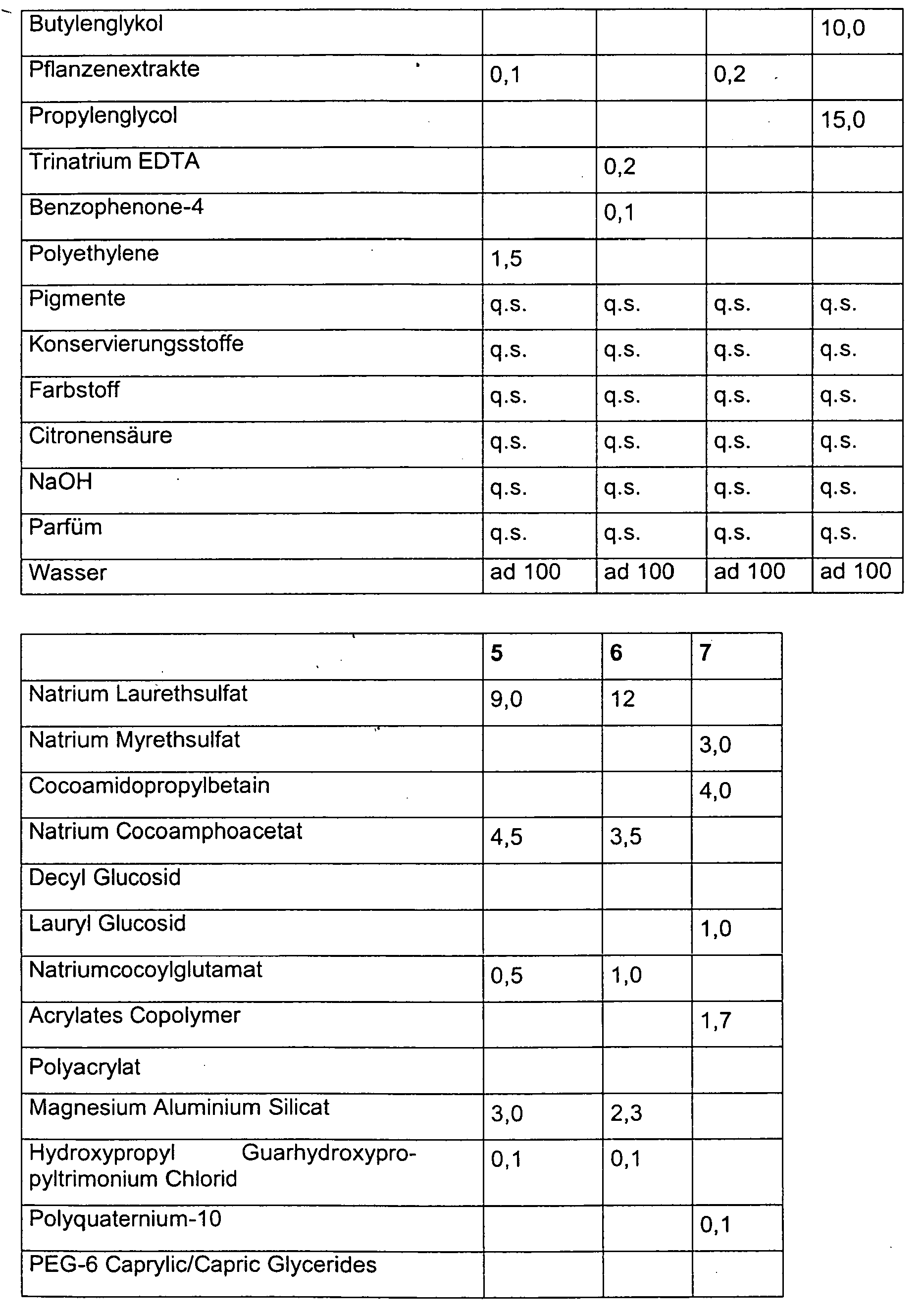

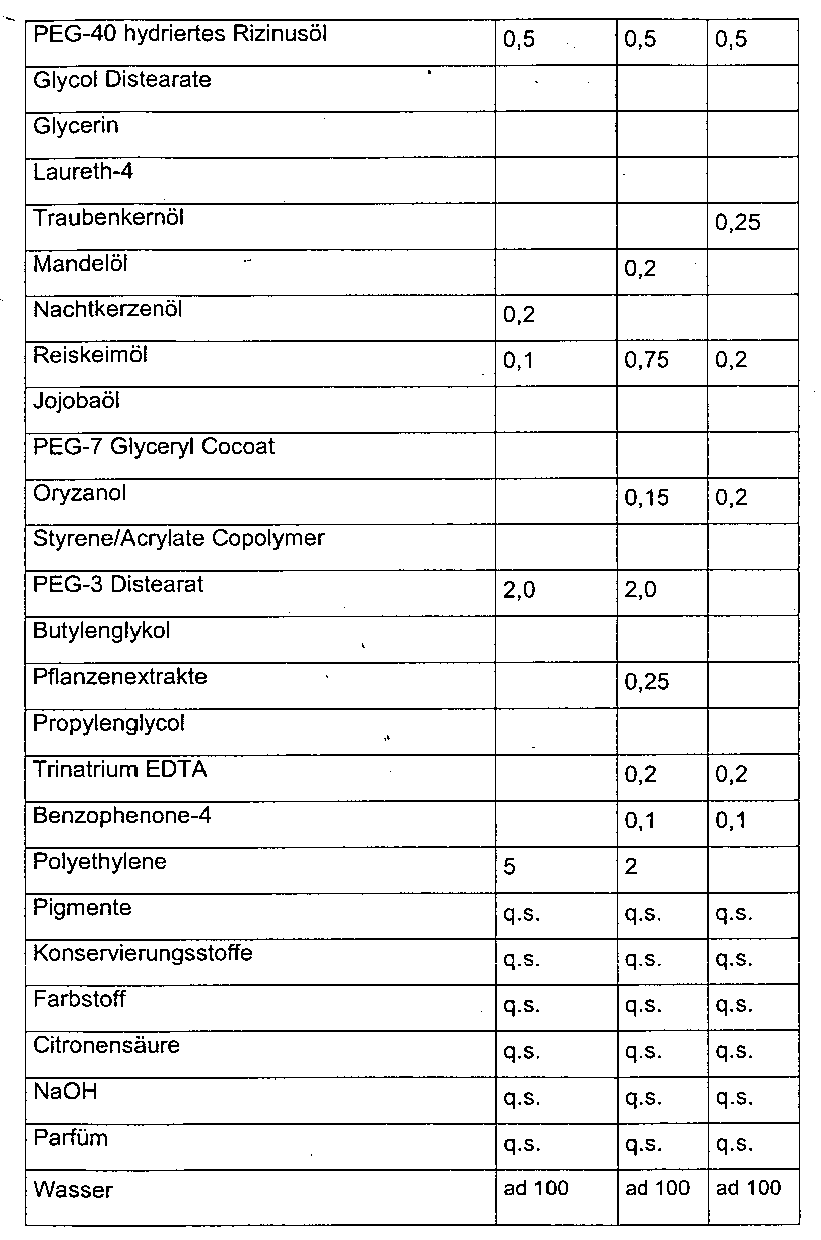

### Tensidhaltige Emulsion:

| | **6** | **7** | **8** | **9** | **10** | **11** |
|---|---|---|---|---|---|---|
| Natriumlaurethsulfat | 11,0 | 6,0 | - | 8,0 | - | - |
| Cocamidopropylbetain | - | 3,5 | - | 2,0 | 2,0 | 4,0 |
| Natrium Myristylethsulfat | - | - | 6,0 | - | 7,0 | 7,0 |
| Alkylpolyglucoside | - | 2,5 | - | - | 2,0 | - |
| Polyacrylat | 0,5 | 0,4 | 0,5 | 0,6 | 0,3 | 0,4 |
| PEG-7 Glyceryl Cocoate | - | - | 1,0 | 1,0 | - | - |
| Paraffinöl | 34,5 | 30,0 | 45,00 | 40,0 | 25,0 | 35,0 |
| Traubenkernöl | 8,4 | 12,0 | - | 5,0 | 15,0 | 10,0 |
| Mandelöl | - | 1,0 | - | 1,0 | - | 1,0 |
| Reiskeimöl | 2,5 | 0,5 | 0,1 | 0,25 | 0,3 | 5,0 |
| Jojobaöl | - | 0,5 | 1,0 | - | 1,0 | - |
| Oryzanol | | 0,05 | 0,1 | 0,5 | | 0,1 |
| Dimethicon | 0,5 | - | 1,0 | 0,5 | - | - |
| Stearin Alkohol | - | - | - | 0,5 | - | - |
| Lanolin Alkohol | 0,3 | - | - | - | - | - |
| Farbstoffe | - | 0,1 | - | - | 0,1 | - |
| Effektpigmente | 0,1 | | 0,2 | - | - | - |
| Reiskleiewachs | | 0,1 | | 0,5 | | 0,2 |
| Glycerin | - | 0,2 | - | 0,5 | - | - |
| Oryzanol | | 0,1 | 0,05 | 0,2 | | 0,1 |
| Pflanzenextrakte | 0,1 | | 0,2 | | | 0,3 |
| Methylparaben | 0,4 | 0,3 | 0,1 | 0,3 | 0,2 | 0,4 |
| Ethylparaben | 0,1 | - | 0,1 | - | 0,1 | - |
| Propylparaben | 0,2 | 0,2 | 0,1 | 0,2 | 0,2 | 0,2 |
| Butylparaben | 0,1 | - | 0,1 | - | 0,1 | - |
| Isobutylparaben | 0,1 | - | 0,1 | - | 0,1 | - |
| Polyethylen | 0,2 | | | 2,0 | | 5,0 |
| Phenoxyethanol | 0,6 | 0,6 | 0,5 | 0,6 | 0,5 | 0,6 |
| Butylhydroxyethanol | 0,1 | 0,1 | 0,1 | 0,1 | 0,1 | - |
| NaOH | q.s. | q.s. | q.s. | q.s. | q.s. | q.s. |
| Parfüm | 1,2 | 0,8 | 1,5 | 1,0 | 1,2 | 1,0 |
| Wasser | ad 100 | ad 100 | ad 100 | ad 100 | ad 100 | ad 100 |

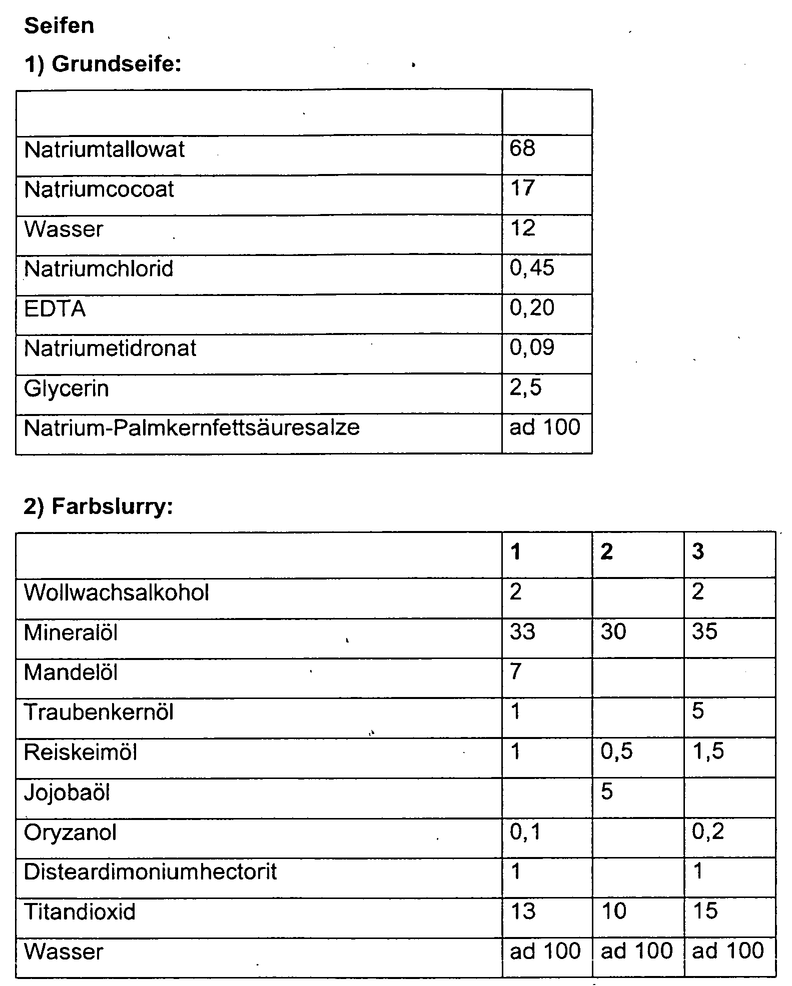

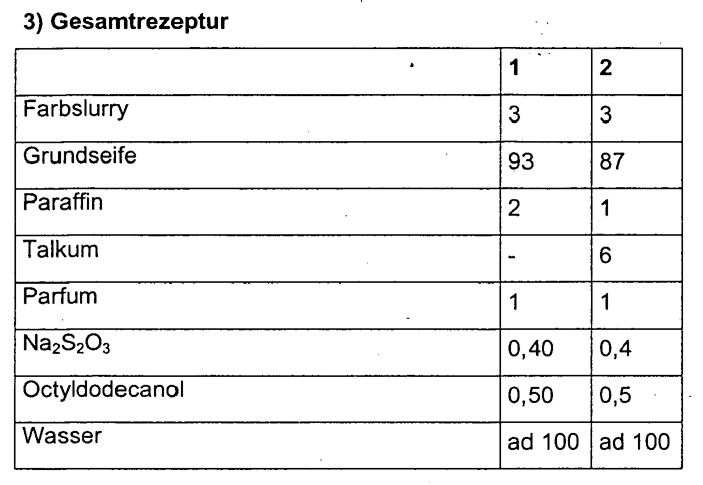

Die Grundseifennudeln werden mit dem Farbslurry und den übrigen Komponenten in einen üblichen Seifenmischer (Schneckenmischer mit Lochsieb) dosiert, durch mehrmaliges Vermischen homogenisiert, über eine Strangpresse ausgetragen, geschnitten und in üblicher Weise zu Stücken verarbeitet.

## Patentansprüche

1. Kosmetische Zubereitung enthaltend Reiskeimöl.

2. Zubereitung nach Patentanspruch 1 **dadurch gekennzeichnet, dass** der Gehalt an Reiskeimöl 0,05 bis 6 Gew.%, bevorzugt 0,2 bis 5 Gew.%, besonders bevorzugt 0,5 bis 2 Gew.% beträgt.

3. Zubereitung nach einem der vorangehenden Patentansprüche **dadurch gekennzeichnet, dass** zusätzlich eine oxidationsempfindliche Substanz enthalten ist.

4. Zubereitung nach einem der vorangehenden Patentansprüche **dadurch gekennzeichnet, dass** die oxidationsempfindliche Substanz in Konzentrationen von mindestens 0,1 Gew.%, bevorzugt 0,5 Gew.%, besonders bevorzugt 1 Gew.%, ganz besonders bevorzugt 5 Gew.% enthalten ist.

5. Zubereitung nach einem der vorangehenden Patentansprüche **dadurch gekennzeichnet, dass** die oxidationsempfindliche Substanz eine Jodzahl von mindestens 80 aufweist.

6. Zubereitung nach einem der vorangehenden Patentansprüche **dadurch gekennzeichnet, dass** die oxidationsempfindliche Substanz ein oxidierbares pflanzliches Öl oder einen pflanzlichen Extrakt, besonders bevorzugt Extrakte der Pflanzenfamilien der Nymphaeaceae, Nelumbonaceae, Fabaceae und/oder Leguminosae enthält.

7. Zubereitung nach einem der vorangehenden Patentansprüche **dadurch gekennzeichnet, dass** sie mindestens 1 Gew.%, bevorzugt mehr als 2 Gew.% oberflächenaktive Substanz, bevorzugt mit einem HLB Wert zwischen 3,8 bis 8 oder größer 10, besonders bevorzugt zwischen 5 und 7 oder größer 12 enthält.

8. Zubereitung nach einem der vorangehenden Patentansprüche **dadurch gekennzeichnet, dass** die oberflächenaktive Substanz aus der Gruppe ethoxylierter Fettalkoholderivate mit einer C-Kettenlänge größer 10 und einem mittleren Ethoxylierungsgrad zwischen 2 und 75, bevorzugt zwischen 2 und 39 besonders bevorzugt zwischen 2 und 5 gewählt wird.

9. Zubereitung nach einem der vorangehenden Patentansprüche **dadurch gekennzeichnet, dass** sie als oberflächenaktive Substanzen anionische Tenside, besonders bevorzugt Alkyl- und/oder Alkylethersulfate in Anteilen von 2-25 Gew.%, bevorzugt 3-20 Gew.% und besonders bevorzugt 7,5-15 Gew.% enthält.

10. Zubereitung nach Patentanspruch 7 bis 9 **dadurch gekennzeichnet, dass** sie weitere anionische, amphotere oder nicht ionische Tenside von 1-15 Gew.%, bevorzugt 3-10 Gew.% und besonders bevorzugt 4-7 Gew.% enthält.

11. Zubereitung nach einem der vorangehenden Patentansprüche **dadurch gekennzeichnet, dass** sie
(a) 1 - 25 Gew.% eines oder mehrerer Alkyl- und/oder Alkylethersulfate,
(b) 0,1-20 Gew.% N-Acylglutamat,
(c) wobei der Gehalt an freien Fettsäuren in den Zubereitungen geringer als 3 Gew.% bevorzugt geringer als 2 Gew. % besonders bevorzugt bis zu 1 Gew.%, aber in allen Fällen mindestens 0,2 Gew.% beträgt,
enthält.

12. Zubereitung nach einem der vorangehenden Patentansprüche **dadurch gekennzeichnet, dass** sie einen Lipidanteil von mindestens 5 Gew.%, bevorzugt mindestens 10 Gew.%, besonders bevorzugt mindestens 15 Gew.% aufweist.

13. Zubereitung nach einem der vorangehenden Patentansprüche **dadurch gekennzeichnet, dass** sie zusätzlich mehr als 0,007 Gew.% Oryzanol, bevorzugt mehr als 0,01 Gew.% Oryzanol enthält.

14. Verwendung von Zubereitungen nach einem der vorangehenden Patentansprüche zur Körperpflege und/oder Körperreinigung.
